# EUROPEAN PATENT APPLICATION

(11) **EP 4 730 127 A1**
(43) Date of publication of application: **22.04.2026**
(21) Application number: 24823116.9
(22) Date of filing: 25.04.2024
(51) Int. Cl.: G06F 9/50, G16C 20/30

(54) **CONTROL DEVICE, CONTROL DEVICE OPERATION METHOD, CONTROL DEVICE OPERATION PROGRAM, AND PHYSICAL PROPERTY PREDICTION SYSTEM**

(30) Priority: 16.06.2023 JP 2023099652
(71) Applicant: FUJIFILM Corporation, Tokyo 106-8620 (JP)
(72) Inventor: SUGIYAMA, Satoshi, Ashigarakami-gun, Kanagawa 258-8577 (JP); TATESHITA, Masakazu, Ashigarakami-gun, Kanagawa 258-8577 (JP); MURAKAMI, Ryoichi, Ashigarakami-gun, Kanagawa 258-8577 (JP); HIKIDA, Yasushi, Ashigarakami-gun, Kanagawa 258-8577 (JP)
(74) Representative: HGF
(86) International application number: PCT/JP2024/016299
(87) International publication number: WO 2024/257494

(57) **Abstract**

A control device including a processor, in which the processor is configured to: accept a request for prediction processing of predicting physical properties of a compound, the request including compound information for specifying the compound to be predicted; derive a job score that is an evaluation value indicating a magnitude of a load of a job corresponding to the accepted request, based on the compound information; and control a scale of a hardware resource for executing a plurality of unprocessed jobs, based on the job score.

## Description

### BACKGROUND OF THE INVENTION

### 1. Field of the Invention

The present disclosure relates to a control device, a method for operating a control device, an operation program for a control device, and a physical property prediction system.

### 2. Description of the Related Art

A physical property prediction device that predicts physical properties of a compound is known (for example, see JP7219374B). The physical property prediction device described in JP7219374B is a server that accepts requests from client terminals of a plurality of users through a network and returns the prediction results to each of the requesting client terminals. A system in which the server provides the application service to the client terminal through the network in this way is also referred to as a cloud system or the like.

### SUMMARY OF THE INVENTION

As in computer systems in many fields, the physical property prediction system includes an installation type in which a prediction program is installed for each computer and a cloud system described in JP7219374B. In the installation type, one user can occupy the hardware resource, but in the cloud system, a plurality of users share the hardware resource. Therefore, in the cloud system, the load on the hardware resource significantly fluctuates depending on the usage status of each user, the content of the processing request, and the like.

For example, in a case where the physical property prediction system is used for drug discovery of pharmaceuticals, the prediction targets of the physical property are compounds that are candidate substances for the pharmaceuticals. In the development process of pharmaceuticals, the number of compounds for which prediction is required significantly fluctuates in each phase of the development process. In an initial phase of the development process of pharmaceuticals, the number of compounds for which prediction is required reaches several hundred thousand, whereas in a final phase thereof, the number of the compounds for which prediction is required is reduced to about several tens. Therefore, depending on the phase undertaken by each of a plurality of users, the number of compounds to be predicted included in the request from the user significantly varies among users. In addition, even in the prediction method for predicting the physical properties of the compound, the load on the hardware resource significantly varies between the prediction method using the machine learning model and the prediction method using the molecular simulation such as the molecular dynamics simulation, thereby significantly fluctuating the load according to which prediction method is selected by the user.

As a general technology of a computer system, a technology such as scaling that controls the scale of the hardware resource in real time with respect to the load fluctuation of the hardware resource is known. In the scaling, for example, the number of prediction processing servers that execute the prediction processing is changed according to the number of prediction processing requests.

However, in the scaling in the related art, the scaling is performed only according to the number of the prediction processing. Therefore, in the physical property prediction of compounds, there have been cases where the appropriate scaling of the hardware resource according to the actual load cannot be performed.

This is because, in physical property prediction of compounds, even in the prediction processing of a single compound, the load on the hardware resource significantly varies depending on the molecular weight of the compound to be predicted. For example, there may be a difference in the molecular weight of several tens of times between a low-molecular-weight compound and a high-molecular-weight compound. The fact that there is a difference of several tens of times in the molecular weight of the compound to be predicted means that the magnitude of the load appears as a difference of several tens of times even in a case where the number of the prediction processing is the same. Therefore, in some cases, appropriate control cannot be performed only by performing scaling according to the number of the prediction processes.

The technology of the present disclosure provides a control device capable of performing appropriate control in physical property prediction of compounds, as compared with a case where scaling of a hardware resource is performed based only on the number of prediction processes, a method for operating a control device, an operation program for a control device, and a physical property prediction system.

A control device according to the technology of the present disclosure including a processor, in which the processor is configured to: accept a request for prediction processing of predicting physical properties of a compound, the request including compound information for specifying the compound to be predicted; derive a job score that is an evaluation value indicating a magnitude of a load of a job corresponding to the accepted request, based on the compound information; and control a scale of a hardware resource for executing a plurality of unprocessed jobs, based on the job score.

The processor may be configured to acquire a correlation between the compound information and the load from a storage in which the correlation has been previously stored, and derive the job score.

The hardware resource may be a server that executes the prediction processing, and the processor may be configured to determine, as the scale, the number of the servers to be assigned to the prediction processing.

The processor may be configured to, when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

The load may be the number of jobs or a job score.

The processor may be configured to set, as queues for registering unprocessed jobs, two queues allowing parallel processing, which are a small-capacity queue in which the small-scale job set is registered and a large-capacity queue in which the large-scale job set is registered, and in the order control, the processor may be configured to: register, among the large-scale job set of the first user, a part of the job set up to the threshold value in the small-capacity queue; register the remaining part of the job set exceeding the threshold value in the large-capacity queue; and register the small-scale job set of the second user in the small-capacity queue.

Separate hardware resources may be assigned to the small-capacity queue and the large-capacity queue.

The processor may be configured to present a waiting time until completion of the job.

A method for operating a control device according to the technology of the present disclosure is a method for operating a control device including a processor, the method including: accepting, by the processor, a request for prediction processing of predicting physical properties of a compound, the request including compound information for specifying the compound to be predicted; deriving, by the processor, a job score that is an evaluation value indicating a magnitude of a load of a job corresponding to the accepted request, based on the compound information; and controlling, by the processor, a scale of a hardware resource for executing a plurality of unprocessed jobs, based on the job score.

An operation program for a control device according to the technology of the present disclosure is an operation program for causing a computer to function as a control device, the operation program causing the computer to execute: a process of accepting a request for prediction processing of predicting physical properties of a compound, the prediction processing request including compound information for specifying the compound to be predicted; a process of deriving a job score that is an evaluation value indicating a magnitude of a load of a job corresponding to the accepted request, based on the compound information; and a process of controlling a scale of a hardware resource for executing a plurality of unprocessed jobs, based on the job score.

A physical property prediction system according to the technology of the present disclosure includes the control device and a prediction processing device that executes the prediction processing as the hardware resource.

Another control device according to the technology of the present disclosure includes a processor, in which the processor is configured to: accept a request for prediction processing of predicting physical properties of a compound; and when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

In another control device, the load may be the number of jobs for each unit of the compound or a job score that is an evaluation value indicating a magnitude of a load determined by the compound.

In another control device, the processor may be configured to set, as queues for registering unprocessed jobs, two queues allowing parallel processing, which are a small-capacity queue in which the small-scale job set is registered and a large-capacity queue in which the large-scale job set is registered, and in the order control, the processor may be configured to: register, among the large-scale job set of the first user, a part of the job set up to the threshold value in the small-capacity queue; register the remaining part of the job set exceeding the threshold value in the large-capacity queue; and register the small-scale job set of the second user in the small-capacity queue.

In another control device, separate hardware resources may be assigned to the small-capacity queue and the large-capacity queue.

In another control device, the processor may be configured to present a waiting time until completion of the job.

A method for operating another control device according to the technology of the present disclosure is a method for operating a control device including a processor, the method including: accepting, by the processor, a request for prediction processing of predicting physical properties of a compound; and when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, executing, by the processor, order control to give priority to a processing order of the small-scale job set of the second user over a processing order of a part of the large-scale job set of the first user.

An operation program for another control device according to the technology of the present disclosure is an operation program for causing a computer to function as a control device, the operation program causing the computer to execute: a process of accepting a request for prediction processing of predicting physical properties of a compound; and when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, a process of executing order control to give priority to a processing order of the small-scale job set of the second user over a processing order of a part of the large-scale job set of the first user.

Another physical property prediction system according to the technology of the present disclosure includes the other control device and a prediction processing device that executes the prediction processing.

According to the present disclosure, in the physical property prediction of compounds, it is possible to perform appropriate control as compared with a case where the scaling of the hardware resource is performed based only on the number of prediction processes.

### BRIEF DESCRIPTION OF THE DRAWINGS

FIG. 1 is a diagram illustrating an outline of a control device of a compound prediction system.
FIG. 2 is a block diagram illustrating an example of a hardware configuration of the control device.
FIG. 3 is a diagram illustrating an example of each process executed by a processor.
FIG. 4 is a diagram illustrating an example of a request acceptance processing.
FIG. 5 is a diagram illustrating an example of job score derivation processing and scaling processing.
FIG. 6 is a flowchart illustrating an overall processing procedure of the control device.
FIG. 7 is a diagram showing a comparative example.
FIG. 8 is a diagram showing types and molecular weights of pharmaceuticals.
FIG. 9 is a diagram illustrating an example of each process executed by a processor according to the second embodiment.
FIG. 10 is a diagram illustrating an example of a request acceptance processing according to the second embodiment.
FIG. 11 is a diagram illustrating an example of job distribution processing.
FIG. 12 is a diagram illustrating another example of job distribution processing.
FIG. 13 is a flowchart illustrating an overall processing procedure of the control device according to a second embodiment.
FIG. 14 is a flowchart illustrating an example of a procedure of the job distribution processing.
FIG. 15 is a diagram illustrating an example of assigning hardware resources to each queue.
FIG. 16 is a diagram illustrating a relationship between each phase in a development process of drug discovery and the number of candidate substances.
FIG. 17 is a diagram illustrating an example of assigning one hardware resource to two queues.
FIG. 18 is an example illustrating an example of a processing order of jobs in a case of FIG. 17.
FIG. 19 is a diagram illustrating an example in which a threshold value is a job score.

### DESCRIPTION OF THE PREFERRED EMBODIMENTS

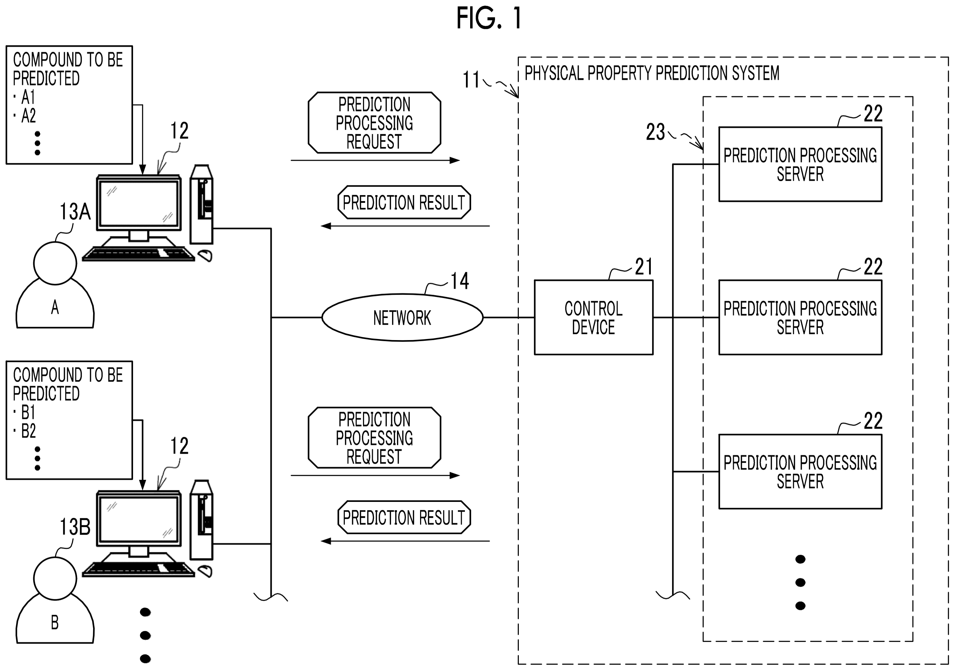

In FIG. 1, the physical property prediction system 11 is a device that predicts the physical properties of a compound and outputs prediction results. The physical property prediction system 11 is a server-type system that distributes prediction results in response to a prediction processing request from the client terminal 12, and is also referred to as a cloud system. The physical property prediction system 11 and the client terminal 12 can communicate with each other through a network 14. The network 14 is a communication network such as a local area network (LAN) and a wide area network (WAN).

The client terminal 12 is operated by the user 13, such as a researcher, who evaluates the physical properties of the compound. The client terminal 12 is, as an example, a personal computer. In a case where the user 13 is distinguished between the user 13 of "A" and the user 13 of "B", 13A and 13B are used as respective reference numerals, but in a case where distinction is not necessary, the user 13 is collectively referred to as the user 13.

The physical property prediction system 11 is used, as an example, in research and development of drug discovery. In research and development of drug discovery, it is important to evaluate absorption, distribution, metabolism, excretion, and toxicity as physical properties of a compound which is a candidate substance for pharmaceuticals. The physical property prediction system 11 is used, as an example, for predicting toxicity among these physical properties. In addition, other physical properties may be predicted, or a plurality of types of physical properties may be predicted.

The access from the client terminal 12 to the physical property prediction system 11 is performed, as an example, through a general-purpose browser. In a case where a uniform resource locator (URL) of the physical property prediction system 11 is input into the browser, the operation screen of the physical property prediction system 11 is distributed from the physical property prediction system 11 to the client terminal 12 by the communication function of the browser. The user 13 inputs the compound information of a compound to be predicted through the operation screen displayed on the browser. In a case where the transmission button is operated, a prediction processing request including the input compound information is transmitted from the client terminal 12 to the physical property prediction system 11. The compound information is information for specifying a compound to be predicted, and is, as an example, data selected from a structural formula, a compositional formula, an amino acid sequence, and the like of the compound.

In addition, the user 13 may be able to select a method of prediction processing, such as a prediction method using a machine learning model or a prediction method using molecular simulation such as molecular dynamics simulation. In this case, the prediction method selected by the user 13 is input through the operation screen and is transmitted to the physical property prediction system 11 as a part of the prediction processing request.

The prediction processing request for the physical property prediction system 11 can include one or more compounds to be predicted. The client terminal 12 can transmit the prediction processing request for one compound one by one, or can collectively transmit the prediction processing requests for a plurality of compounds. In FIG. 1, an example is shown in which the user 13A inputs, as prediction targets, a plurality of compounds such as A1, A2, ... into the client terminal 12, and the client terminal 12 collectively transmits prediction processing requests for the plurality of input compounds. An example is shown in which the user 13B inputs, as prediction targets, a plurality of compounds such as B1, B2, ... into the client terminal 12, and the client terminal 12 collectively transmits prediction processing requests for the plurality of input compounds. The prediction processing request is an example of a "request" according to the technology of the present disclosure.

The physical property prediction system 11 includes a control device 21 and a plurality of prediction processing servers 22. The prediction processing server 22 is an example of a "prediction processing device" according to the technology of the present disclosure. The control device 21 and the prediction processing server 22 are connected to each other, as an example, through a LAN in a communicable manner. The prediction processing server 22 executes prediction processing of predicting physical properties of a compound and outputs prediction results. The prediction processing server 22 is, for example, a computer on which a prediction processing program for predicting physical properties of a compound based on compound information is installed. The prediction processing program includes a program for performing molecular simulation, in addition to a program using a machine learning model. The prediction processing server 22 configures a hardware resource 23 for executing a job. The plurality of prediction processing servers 22 may be configured by physically independent computers, or may be configured by a plurality of virtual servers that are virtually configured by installing a plurality of operating systems (OS) on one computer.

The control device 21 receives the prediction processing request from the client terminal 12 and distributes the prediction result output by the prediction processing server 22 to the requesting client terminal 12. In addition, as will be described later, the control device 21 has a function of controlling the scale of the hardware resource 23, and changes the scale of the hardware resource 23 in real time according to the processing status or the like.

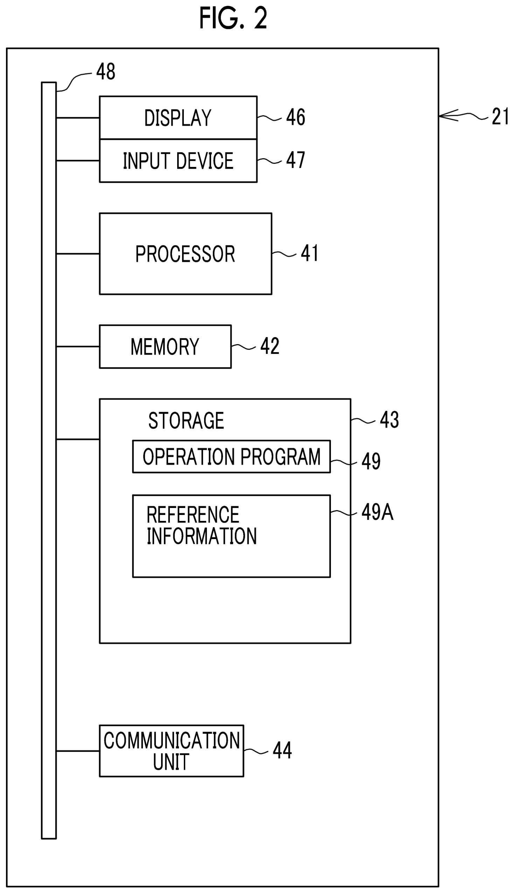

In FIG. 2, the computer constituting the control device 21 includes a processor 41, a memory 42, a storage 43, a communication unit 44, a display 46, and an input device 47. These units are connected to each other through a bus line 48. The processor 41 is, as an example, a central processing unit (CPU).

The storage 43 is a hard disk drive that is provided in the computer constituting the control device 21 or is connected to the computer through a cable or a network. Alternatively, the storage 43 is a disk array in which a plurality of hard disk drives are mounted. It should be noted that a solid state drive may be used instead of the hard disk drive. The storage 43 stores a control program such as an operating system, various application programs including an operation program 49, various types of data associated with these programs, and the like. The various types of data include reference information 49A that is referred to by the operation program 49 to execute processing.

The memory 42 is a work memory for the processor 41 constituted by a CPU to execute processing. The processor 41 integrally controls the units of the computer by loading the programs stored in the storage 43 into the memory 42 and executing processing compliant with the programs. The operation program 49 is an application program for causing the computer to function as the control device 21, and is an example of an "operation program for a control device" according to the technology of the present disclosure.

The communication unit 44 is a network interface that controls transmission of various types of information via the network. For example, communication with the client terminal 12 and the prediction processing server 22 is performed through the communication unit 44. The display 46 displays various screens for operating the control device 21. The control device 21 receives an input of an operation instruction from the input device 47 through various screens.

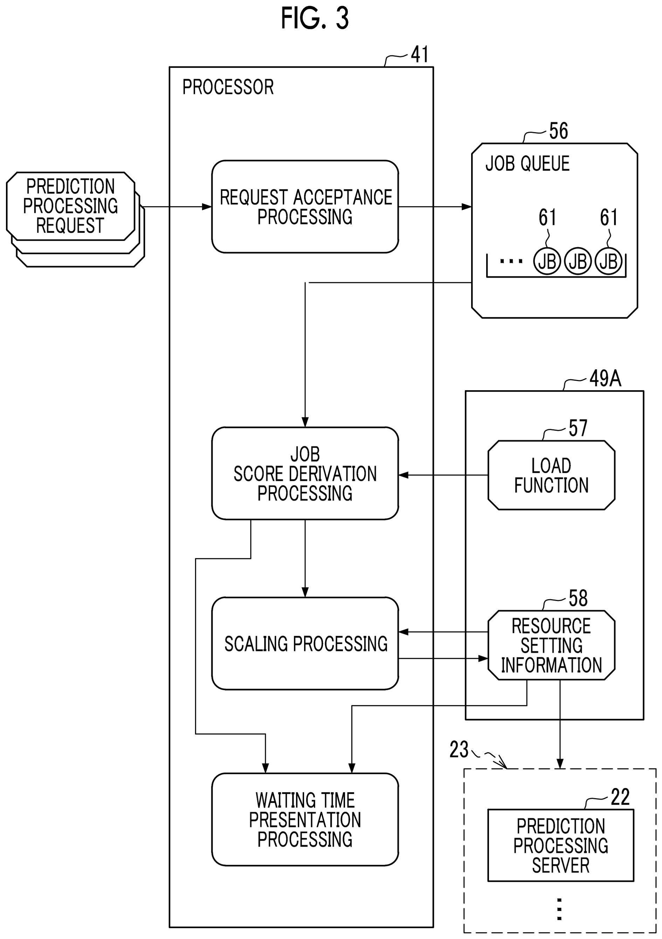

As shown in FIG. 3, in a case where the processor 41 executes the operation program 49, the processor 41 executes the request acceptance processing, the job score derivation processing, the scaling processing, and the waiting time presentation processing.

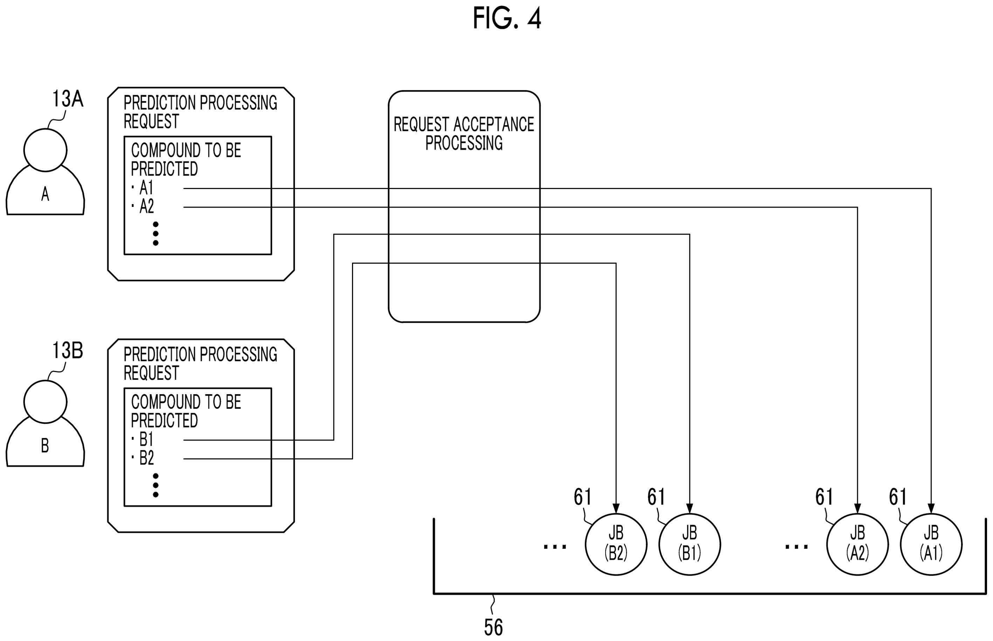

The request acceptance processing is processing of accepting the prediction processing request from the client terminal 12. As shown in FIG. 4, in a case where the prediction process request is accepted, the processor 41 registers the accepted prediction processing request in the job queue 56 as a job of a unit of the compound. The job 61 is a unit in which the prediction processing server 22 executes processing, and the job queue 56 is a queue that holds unprocessed jobs in a waiting state. In the present example, one job 61 is a process of predicting physical properties of one compound, and the number of jobs 61 coincides with the number of compounds to be predicted.

In the job queue 56, jobs corresponding to the accepted prediction processing requests are registered in the acceptance order in which the prediction processing requests are accepted. The job 61 registered in the job queue 56 is read out from the job queue 56 by the prediction processing server 22 in the order of registration or in the acceptance order of the prediction processing request, and is executed.

The example shown in FIG. 4 is an example in which the acceptance order of the prediction processing request from the user 13A is earlier than that of the prediction processing request from the user 13B. In the job queue 56, the job 61 (B1, B2, ...) of the user 13B is registered after the job 61 (A1, A2, ...) of the user 13A.

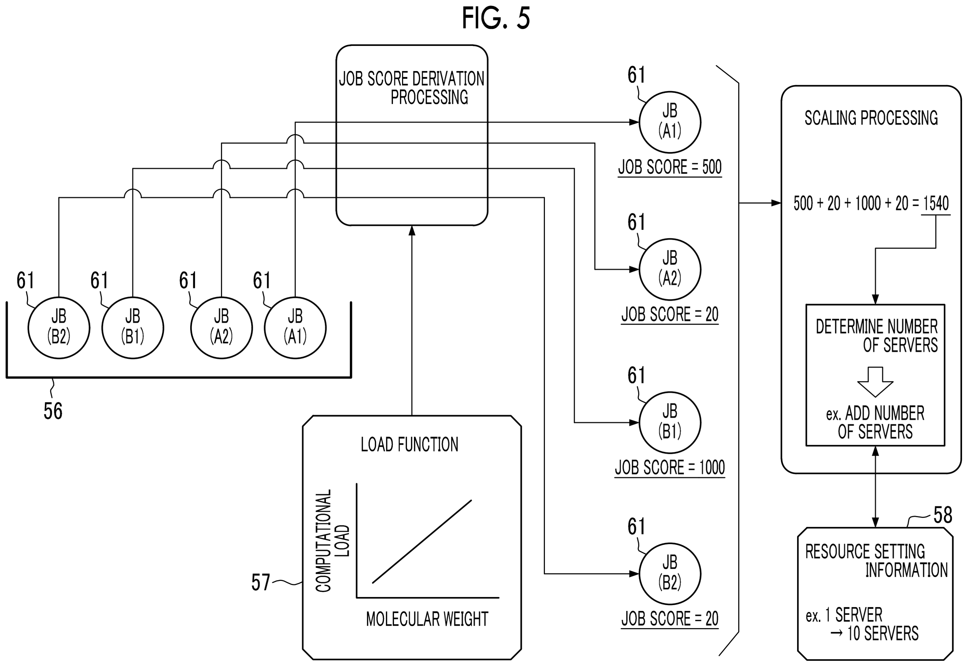

As shown in FIG. 5, the processor 41 derives the job score based on the compound information in the job score derivation processing. The job score is an evaluation value indicating the magnitude of the load of the job 61 corresponding to the accepted prediction processing request. The load of the job 61 is a load of the computational processing (hereinafter, referred to as a computational load) that is borne by the prediction processing server 22 in a case where the prediction processing is executed, and is an example of a "load" according to the technology of the present disclosure. The computational load of the prediction processing and the molecular weight of the compound have a correlation in which the larger the molecular weight is, the larger the computational load is. The reference information 49A includes the load function 57 which is an example of such a correlation. The load function 57 may be in a form of an arithmetic expression or a form of table data.

In the job score derivation processing, the processor 41 extracts the molecular weight from the compound information of the compound to be predicted of the job 61, and derives the computational load corresponding to the extracted molecular weight with reference to the load function 57. Then, the processor 41 records the derived computational load as a job score in the memory 42, the storage 43, or the like. The processor 41 derives a job score for each job 61. In the example shown in FIG. 5, as an example, the job score of the job 61 of the compound A1 of the user 13A is "500", and the job score of the job 61 of the compound A2 is "20". In addition, the job score of the job 61 of the compound B1 of the user 13B is "1,000", and the job score of the job 61 of the compound B2 is "20". As described above, even in the prediction processing of predicting the physical properties of one compound, there is a difference in the job score depending on the molecular weight of the compound. As described above, the processor 41 acquires the load function 57, which is an example of the correlation between the compound information and the computational load, from the storage 43 in which the load function 57 is stored in advance, and derives the job score.

In addition, even with the same molecular weight, the computational load changes depending on whether a prediction method using a machine learning model or a prediction method using molecular simulation is used. Therefore, the load function 57 may be set for each prediction method. In this case, for example, the processor 41 reads out the prediction method selected by the user 13 from the prediction processing request, and derives the job score by using the load function 57 in accordance with the selected prediction method.

In the scaling processing, the processor 41 totals the job scores of the unprocessed jobs 61 registered in the job queue 56. In the example shown in FIG. 5, the total value of the job scores is "500" + "20" + "1,000" + "20" = "1,540". Then, the processor 41 controls the scale of the hardware resource 23 based on the total value. The scale is, as an example, the number of prediction processing servers 22 to be assigned to the prediction processing, and the processor 41 determines the number of prediction processing servers 22 corresponding to the total value of the job scores. The individual processing capacity of the prediction processing server 22 is determined by the specification, and the processing capacity is, for example, a processing time per unit job score. The overall processing capacity of the hardware resource 23 is increased in a case where the number of prediction processing servers 22 is increased, and is decreased in a case where the number of prediction processing servers 22 is decreased.

In a case where the number of prediction processing servers 22 is constant, as the total value of the job scores increases, the computational load borne by one prediction processing server 22 in the hardware resource 23 increases. In this case, the processing time until completion of all the unprocessed jobs 61 is increased. In addition, in a case where the total value of the job scores is small and the number of prediction processing servers 22 is too large, the hardware resource 23 is over-specified. The processor 41 determines, as an example, the number of prediction processing servers 22 such that the computational load borne by one prediction processing server 22 does not exceed a preset range, based on the total value of the job scores.

The processor 41 records the determined number of prediction processing servers 22 in the resource setting information 58. For example, identification information of the assigned prediction processing server 22 is recorded in the resource setting information 58.

In the waiting time presentation processing, the processor 41 calculates the processing time of the unprocessed job 61 based on the total value of the job scores of the unprocessed jobs 61 registered in the job queue 56 and the processing capacity corresponding to the scale of the hardware resource 23. Then, the processor 41 calculates the waiting time for each user 13 based on the processing time of the unprocessed job 61. Since the job 61 is executed in the order registered in the job queue 56, the waiting time of the user 13 whose acceptance order is late is longer than that of the user 13 whose acceptance order is early. In the examples shown in FIGS. 4 and 5, the waiting time of the user 13B is longer than that of the user 13A. Of course, in a case of calculating the waiting time of the user 13B, the processor 41 considers the processing time of the job 61 of the user 13A whose acceptance order is earlier than the user 13B.

The processor 41 distributes the calculated waiting time to the client terminal 12 of the requesting user 13. As a result, the processor 41 presents, to the user 13, the waiting time until completion of the job 61 for each user 13.

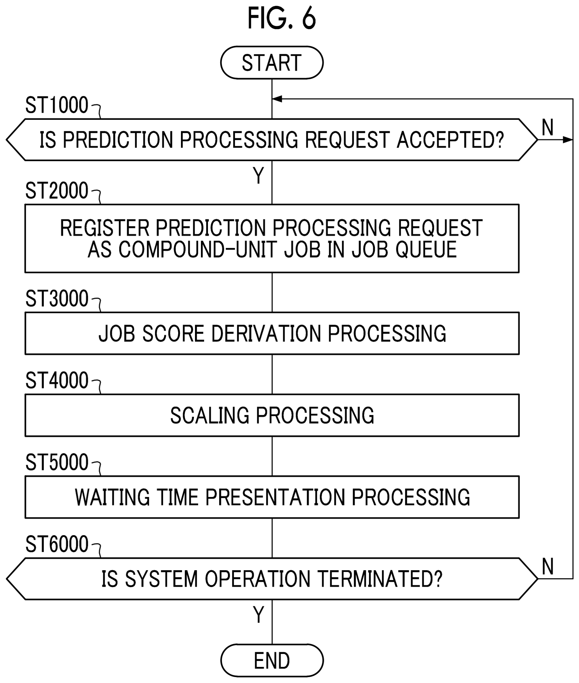

An action of the above configuration will be described with reference to the flowchart illustrated in FIG. 6. As shown in FIG. 6, in a case where the physical property prediction system 11 is activated, in Step ST1000, the processor 41 waits for the prediction processing request. In a case where the prediction processing request is accepted (Y in Step ST1000), the processor 41 proceeds to Step ST2000.

In Step ST2000, the processor 41 executes the request acceptance processing as shown in FIG. 4. In the request acceptance processing, the processor 41 registers the accepted prediction processing request as the job 61 in the unit of the compound in the job queue 56.

In Step ST3000, the processor 41 executes the job score derivation processing as shown in FIG. 5. In the job score derivation processing, the processor 41 derives the total value of the job scores of the unprocessed jobs 61 based on the compound information included in the prediction processing request and the load function 57.

In Step ST4000, the processor 41 executes the scaling processing as shown in FIG. 5. In the scaling processing, the processor 41 controls the scale of the hardware resource 23 by determining the number of prediction processing servers 22 based on the total value of the job scores.

In Step ST5000, the processor 41 executes the waiting time presentation processing. The processor 41 calculates a waiting time for each user 13 based on the job score of the unprocessed job 61, the processing capacity of the hardware resource 23, and the acceptance order of the job 61 for each user 13, and presents the calculated waiting time to the user 13.

The processor 41 repeats the processes of Step ST1000 to Step ST5000 until the completion of the operation of the physical property prediction system 11 (Step ST6000).

As described above, in the control device 21 according to the technology of the present disclosure, the processor 41 is configured to: accept a request for prediction processing of predicting physical properties of a compound, the prediction processing request including compound information for specifying the compound to be predicted; derive a job score that is an evaluation value indicating the magnitude of the load of the job 61 corresponding to the accepted request, based on the compound information; and control a scale of a hardware resource 23 for executing a plurality of unprocessed jobs 61, based on the job score. Therefore, in the physical property prediction of compounds, it is possible to perform appropriate control as compared with a case where the scaling of the hardware resource is performed based only on the number of prediction processes.

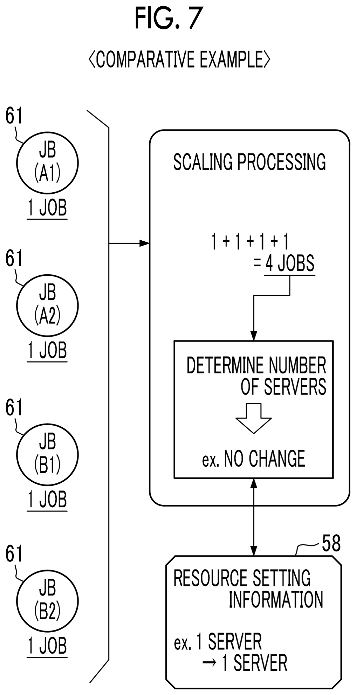

For example, as in the comparative example shown in FIG. 7, in a case where the scaling processing is performed only with the number of the job 61 without using the job score, the difference in the load for each compound is not taken into consideration. In a case where there are four unprocessed jobs 61, the number of prediction processing servers 22 is determined according to the number of jobs without considering the difference in load for each job 61. On the other hand, according to the technology of the present disclosure, even in a case where the same number of jobs 61 are accepted, the processor 41 performs the scaling processing in consideration of the difference in the load for each compound, such as the difference in the load due to the molecular weight of the compound. Therefore, it is possible to appropriately perform scaling of the hardware resource 23 corresponding to the actual load. As a result, it is possible to suppress the variation in the processing time due to the difference in the load for each compound of the unprocessed job 61.

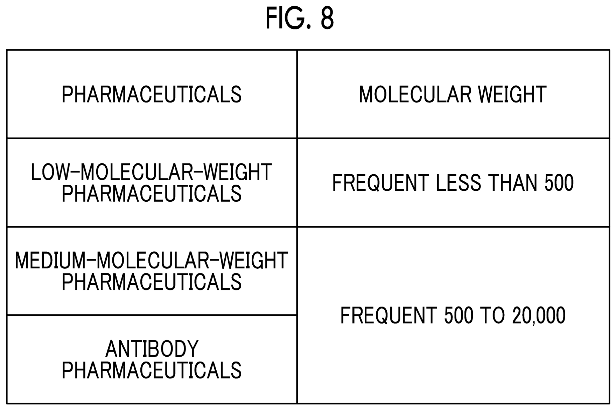

As shown in FIG. 8, the types of pharmaceuticals include not only a large number of low-molecular-weight pharmaceuticals having a molecular weight of about 500 or less but also medium-molecular-weight pharmaceuticals or antibody pharmaceuticals having a molecular weight of 500 to 20,000. In a case where the molecular weights of the low-molecular-weight pharmaceuticals and the antibody pharmaceuticals are compared, there is a difference of 40 times or more in a case where the difference is large. As shown in the load function 57, since the difference in the molecular weight appears as the difference in the computational load of the prediction processing, the difference in the load of one job 61 may also be 40 times or more. In recent years, drug discovery of medium-molecular-weight pharmaceuticals or antibody pharmaceuticals has been actively performed, and it is considered that such a tendency will continue in the future. Therefore, the technology of the present disclosure of scaling the hardware resource with a focus on the compound information is particularly effective in such a field.

In addition, in the above-described embodiment, the processor 41 acquires the correlation between the compound information and the load (as an example, the load function 57) from the storage 43 in which the correlation is stored in advance, and derives the job score. Therefore, processing can be performed in a short time as compared with a case where the correlation is obtained each time.

In addition, in the above-described embodiment, the hardware resource 23 is the prediction processing server 22 (an example of a server according to the technology of the present disclosure) that executes the prediction processing, and the processor 41 determines the number of prediction processing servers 22 to be assigned to the prediction processing as the scale. In the scaling processing of the hardware resource 23, since it is common to change the number of prediction processing servers 22, it is easy to introduce the method as compared with other methods.

### [Second embodiment]

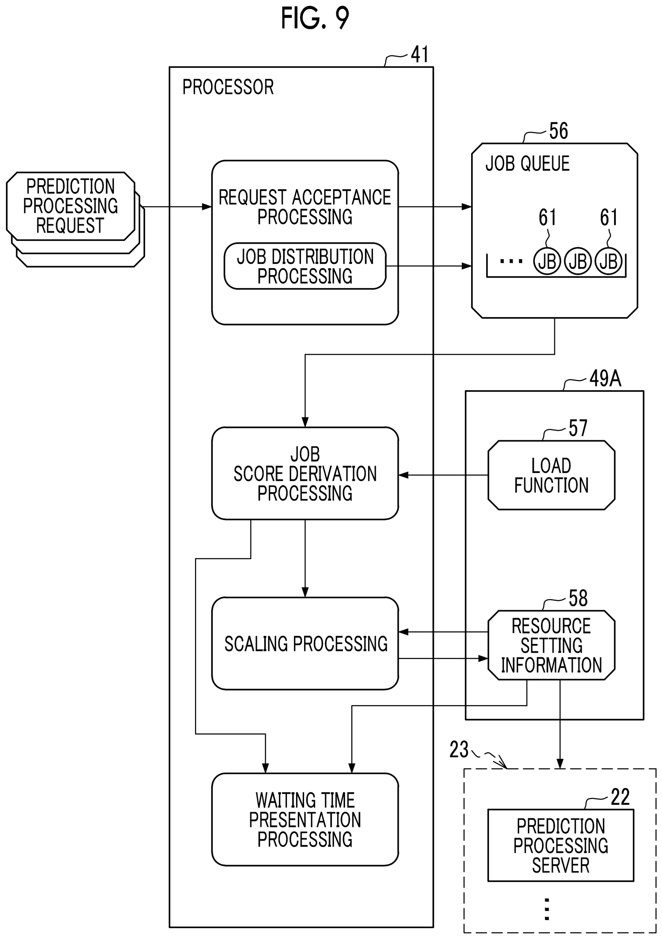

The control device 21 according to the second embodiment shown in FIGS. 9 to 15 is a form in which, in a case where another user 13 performs a small-scale prediction processing request with a relatively small number of requests after a user 13 who performs a large-scale prediction processing request with a relatively large number of requests, the small-scale prediction processing request is preferentially processed before the completion of all the large-scale prediction processing requests under a certain condition.

As shown in FIG. 9, in the second embodiment, the processor 41 executes job distribution processing in the request acceptance processing. Since the second embodiment is the same as the first embodiment except for the job distribution processing, the descriptions other than job distribution processing are omitted.

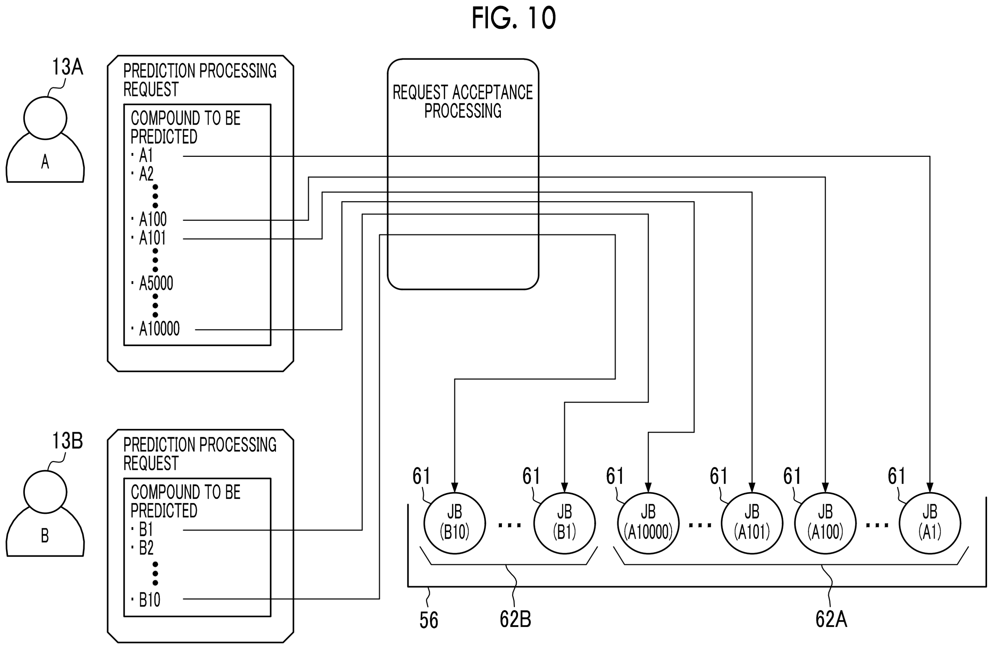

As shown in FIG. 10, as an example, a case where prediction processing requests for 10,000 compounds are continuously performed from the user 13A and then prediction processing requests for 10 compounds are continuously made from another user 13B is considered. The user 13A is an example of a "first user", and the user 13B is an example of a "second user". The processor 41 registers each of the prediction processing requests for 10,000 compounds of the user 13A and the prediction processing requests for 10 compounds of the user 13B in the job queue 56 in the order in which the prediction processing requests are accepted, as the job 61 in unit of the compound. Hereinafter, the series of jobs 61 corresponding to the continuous requests will also be referred to as a job set 62. In addition, in a case where the job sets 62 of the user 13A and the user 13B are distinguished from each other, a series of jobs 61 of the user 13A is referred to as a job set 62A, and a series of jobs 61 of the user 13B is referred to as a job set 62B.

Here, the continuous requests is typically a request for collectively performing prediction processing of a plurality of compounds designated by one user 13 after designating the plurality of compounds to be predicted, as shown in FIG. 10. In addition, a case where a plurality of requests for prediction processing, in which one or more compounds are designated as prediction targets, are separately transmitted within a range of a preset time may also be included in the continuous requests. The range of time for being set as the continuous requests may be defined as a time of 5 minutes or less or 10 minutes or less, or a plurality of requests transmitted within a period in which the user 13 is logged in to the system may be regarded as the continuous requests. That is, the series of jobs 61 or the job set 62 is a collection of a plurality of jobs 61 satisfying the certification conditions of such continuous requests.

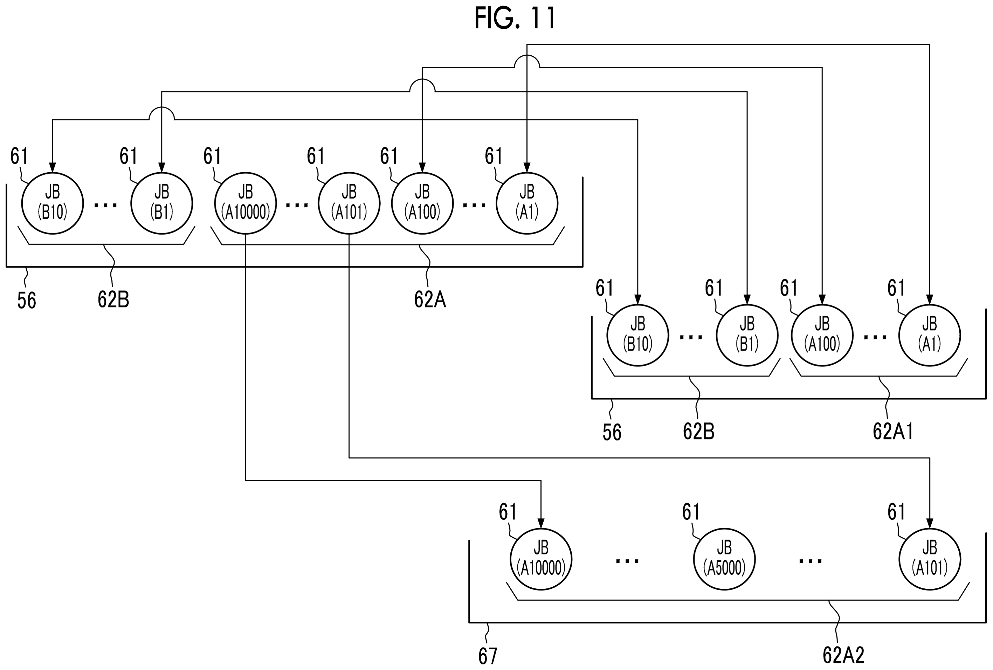

As shown in FIG. 11, the processor 41 of the control device 21 according to the second embodiment executes job distribution processing of distributing the job set 62A and the job set 62B registered in the job queue 56. As shown in FIG. 11, as an example, the processor 41 sets, as queues for which unprocessed jobs 61 are registered, two queues of a small-capacity queue 66 and a large-capacity queue 67 in addition to the job queue 56. In the present example, the job queue 56 is a primary queue registered before the job distribution processing, and the small-capacity queue 66 and the large-capacity queue 67 are secondary queues registered after the job distribution processing.

The small-capacity queue 66 is a queue in which the small-scale job set is registered, and the large-capacity queue 67 is a queue in which the large-scale job set is registered. The small-scale job set is a queue in which a job set 62 having a load equal to or less than a preset threshold value is registered. The small-scale job set also includes a case where the number of jobs 61 is one. The large-capacity queue 67 is a queue in which the job set 62 having a load exceeding a preset threshold value is registered.

The load is, for example, the number of jobs 61, and the threshold value is, as an example, 100. The job set 62A consisting of 10,000 jobs 61 of the user 13A shown in FIG. 10 is an example of a large-scale job set, and the job set 62B consisting of 10 jobs 61 of the user 13B is an example of a small-scale job set. That is, the job set 62 that can be registered in the small-capacity queue 66 has a restriction on the load.

As shown in FIG. 11, the processor 41 executes job distribution processing of distributing the job 61 registered in the job queue 56 which is a primary queue, to the small-capacity queue 66 and the large-capacity queue 67. In the example shown in FIG. 11, the job set 62A1 of the user 13A and the job set 62B of the user 13B are registered in the small-capacity queue 66. The job set 62A1 is a part of the job set 62A including the 10,000 jobs 61 of the user 13A shown in FIG. 10, and specifically includes 100 series of jobs 61. In addition, the job set 62B includes 10 series of jobs 61 of the user 13B shown in FIG. 10. As described above, in the small-capacity queue 66, the maximum value of the number of jobs 61 that are continuously processed for the same requesting user 13 is set to 100.

On the other hand, in FIG. 11, a job set 62A2 including 9,900 jobs 61 for the requesting user 13A has been registered in the large-capacity queue 67. The job set 62A2 is a remainder of the job set 62A (including 10,000 jobs 61) shown in FIG. 10, excluding the job set 62A1 (including 100 jobs 61) registered in the small-capacity queue 66.

As described above, the processor 41 executes the job distribution processing and distributes the job 61 registered in the job queue 56 shown in FIG. 10 to the small-capacity queue 66 and the large-capacity queue 67. As a result, a small-scale job set, which is the job set 62 having a unit of 100 or less as the threshold value, is registered in the small-capacity queue 66. For the large-scale job set exceeding the threshold value, such as the job set 62A of the user 13A, the processor 41 registers, among the large-scale job set, a part of the job set 62A1 up to the threshold value in the small-capacity queue 66. Then, the remaining job set 62A2 exceeding the threshold value is registered in the large-capacity queue 67. On the other hand, the processor 41 registers the job set 62B, which is the small-scale job set of the user 13B, in the small-capacity queue 66 in the processing order after the job set 62A1 of the user 13A.

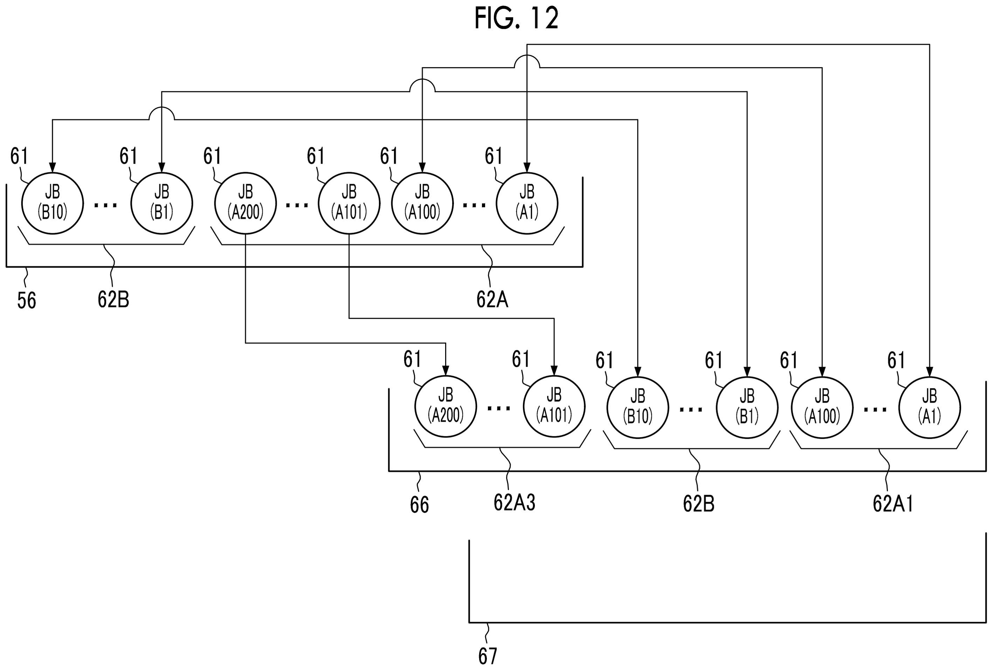

In addition, as shown in FIG. 12, in a case where the job set 62A of the user 13A includes 200 jobs 61 instead of 10,000 jobs 61, the remainder excluding the 100 jobs 62A to be registered in the small-capacity queue 66 is the job set 62A3 including 100 jobs 61. In this case, since the job set 62A3 is also equal to or less than the threshold value, the job set 62A3 is registered in the small-capacity queue 66. As an example, the registration order of the remaining job set 62A3 in the small-capacity queue 66 is after that of the job set 62B of the user 13B, in a case where there is a job set 62 of one or more other users 13 whose acceptance order is later than that of the user 13A and which has a load equal to or less than the threshold value. As a result, in the registration order of the small-capacity queue 66, the 100 job sets 62A1 of the user 13A whose acceptance order is earlier are registered, the 10 job sets 62B of the user 13B whose acceptance order is later than the user 13A are registered, and then the job set 62A3 of the user 13A is registered.

As described above, the processor 41 executes the order control to give priority to a processing order of the job set 62B corresponding to the small-scale job set of the user 13B over that of the job set 62A2 which is a part of the job set 62A corresponding to the large-scale job set of the user 13A.

In addition, the small-capacity queue 66 and the large-capacity queue 67 can perform parallel processing for the prediction processing. For example, one prediction processing server 22 can execute the unprocessed job 61 registered in the small-capacity queue 66 in order, and another prediction processing server 22 can execute the unprocessed job 61 registered in the large-capacity queue 67 in order regardless of the progress status of the small-capacity queue 66.

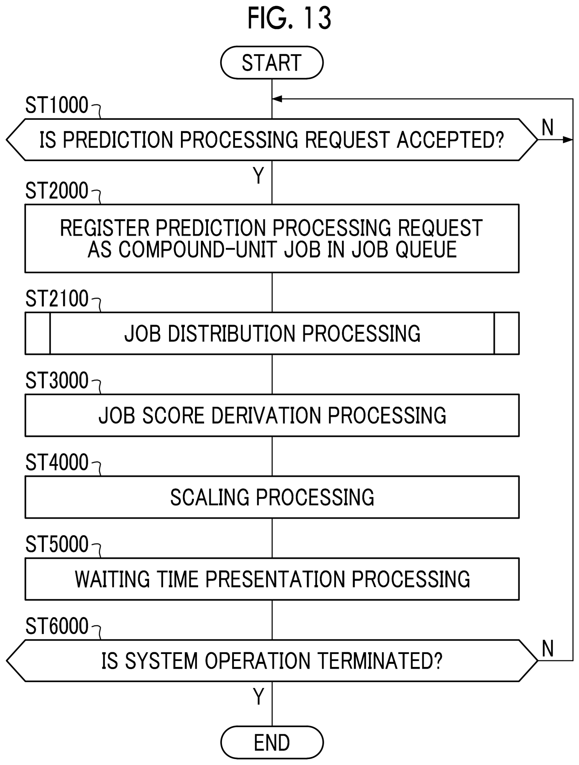

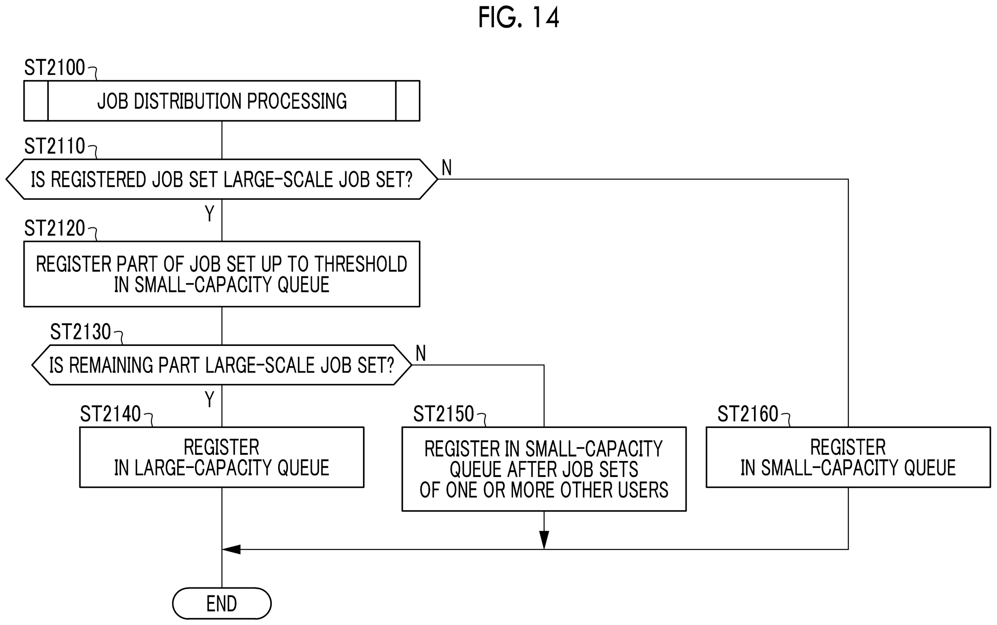

In a case where the processing procedure of such a second embodiment is summarized in a flowchart, the flowchart is as shown in FIGS. 13 and 14. The flowchart shown in FIG. 13 shows an overall processing procedure, and a difference from the flowchart of the first embodiment shown in FIG. 6 is whether or not job distribution processing of Step ST2100 is included. As shown in FIG. 13, in Step ST2000, the processor 41 registers the prediction processing request accepted as shown in FIG. 10 in the job queue 56 as the job 61 in a unit of the compound, and then executes the job distribution processing in Step 2100.

Then, in Step ST2100, the processor 41 executes the process shown in FIG. 14. In Step ST2110, the processor 41 determines whether or not the job set 62 registered in the job queue 56 is the large-scale job set based on the threshold value. In a case where the job set 62 is equal to or less than the threshold value, the processor 41 determines that the job set 62 is the small-scale job set (N in Step ST2110), and proceeds to Step ST2160. Then, in Step ST2160, the processor 41 registers the registered job set 62 in the small-capacity queue 66. On the other hand, in a case where the job set 62 exceeds the threshold value, the processor 41 determines that the job set 62 is the large-scale job set (Y in Step ST2110), and proceeds to Step ST2120. In the case of the present example, since the threshold value is the number of jobs, as shown in FIG. 10, in a case where the number of jobs 61 is 10,000 as in the job set 62A of the user 13A, it is determined that the job set is a large-scale job set. Then, in a case where the number of jobs 61 is 10 as in the job set 62B of the user 13B, it is determined that the job set is a small-scale job set.

In Step ST2120, the processor 41 registers, among the large-scale job set such as the job set 62A of the user 13A, a part of the job set 62A1 up to the threshold value in the small-capacity queue 66.

In Step ST2130, the processor 41 determines whether or not the remaining job set 62 excluding the part of the job set 62 registered in the small-capacity queue 66 among the large-scale job set, is the large-scale job set exceeding the threshold value. In a case where the remaining job set 62 is a large-scale job set exceeding the threshold value of 100 (Y in Step ST2130), as in the job set 62A2 shown in FIG. 11, the processor 41 proceeds to Step ST2140 and registers the remaining job set 62A2 in the large-capacity queue 67. On the other hand, in a case where the remaining job set 62 is a small-scale job set equal to or less than threshold values of 100, as in the job set 62A3 shown in FIG. 12 (N in Step ST2130), the processor 41 proceeds to Step ST2150 and registers the remaining job set 62 in the small-capacity queue 66.

In Step ST2150, the processor 41 registers the remaining small-scale job set, which is a part of the large-scale job set such as the job set 62A3, after the job set 62 of one or more other users 13. As an example, as shown in FIG. 12, the processor 41 registers the job set 62A3 of the user 13A after the job set 62B of the user 13B.

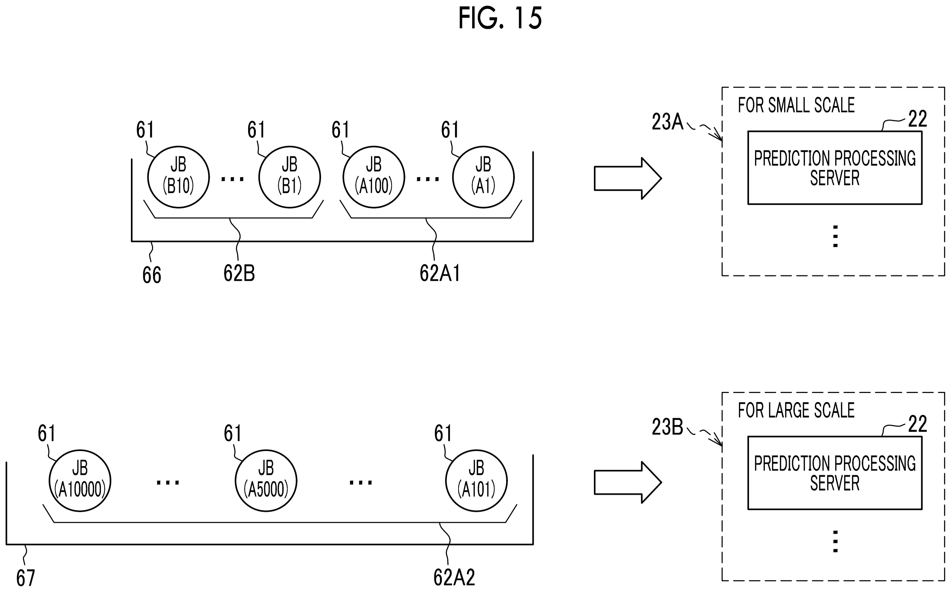

As described above, in the control device 21, the prediction processing is executed on the job sets 62 registered in the small-capacity queue 66 and the large-capacity queue 67 as shown in FIG. 15.

As shown in FIG. 15 as an example, a hardware resource 23A for small-capacity queue is assigned to the small-capacity queue 66, and a hardware resource 23B for a large-capacity queue is assigned to the large-capacity queue 67. One or more prediction processing servers 22 are assigned to each of the hardware resources 23A and 23B. Then, the job 61 registered in the small-capacity queue 66 is processed in the order of registration by the hardware resource 23A for the small-capacity queue. As described above, the jobs 61 registered in the small-capacity queue 66 and the large-capacity queue 67 are processed in parallel.

The job set 62B, which is the small-scale job set of the user 13B, is later in the acceptance order of the prediction processing request than the job set 62A, which is the large-scale job set of the user 13A. However, the job set 62B of the user 13B is registered after the job set 62A1 corresponding to the small-scale job set of a part of the job set 62A of the user 13A in the small-capacity queue 66. The small-capacity queue 66 and the large-capacity queue 67 in which the job set 62A2 corresponding to the remaining large-scale job set of the user 13A is registered are processed in parallel. Therefore, the processing of the job set 62B of the user 13B is started without waiting for the completion of the job set 62A2 of the user 13A registered in the large-capacity queue 67.

In the second embodiment, since the waiting time presentation processing is executed in the same manner as in the first embodiment, each user 13 can confirm the waiting time until completion of the job 61 of each user 13.

As described above, in the control device 21 according to the second embodiment, when, after accepting a request from the user 13A (an example of a first user), accepting a request from the user 13B (an example of a second user) different from the user 13A, in a case where a series of jobs corresponding to continuous requests from the user 13A is a large-scale job set having a load exceeding a preset threshold value and a series of jobs corresponding to continuous requests from the user 13B is a small-scale job set having a load equal to or less than the threshold value, order control to give priority to a processing order of the small-scale job set of the user 13B over a processing order of a part of the large-scale job set of the user 13A is executed.

Therefore, even in a case where a large-scale prediction processing request is input into the physical property prediction system 11, a small-scale prediction processing request to be input thereafter is not put on hold until all the large-scale prediction processing requests that precede the small-scale prediction processing request are completed.

The user 13 who performs a large-scale prediction processing request somewhat assumes that the processing may take time, considering the scale of the prediction processing request input by the user 13. On the other hand, the user 13 who performs a small-scale prediction processing request tends to expect that the processing does not take much time because the scale of the prediction processing request input by the user 13 is small. Therefore, the user 13 who performs a small-scale prediction processing request may have a greater psychological burden on the increase in waiting time than the user 13 who performs a large-scale prediction processing request. Therefore, in the control device 21 of the present disclosure, in a case where a large-scale prediction processing request and a small-scale prediction processing request compete with each other, order control is performed to give priority to the processing order of the small-scale prediction processing request under a certain condition such that the waiting time is set to be appropriate for the scale of the prediction processing request of the user 13. As a result, the waiting time of the user 13 who performs a small-scale prediction processing request is suppressed from being increased, and as a result, the psychological burden is also reduced.

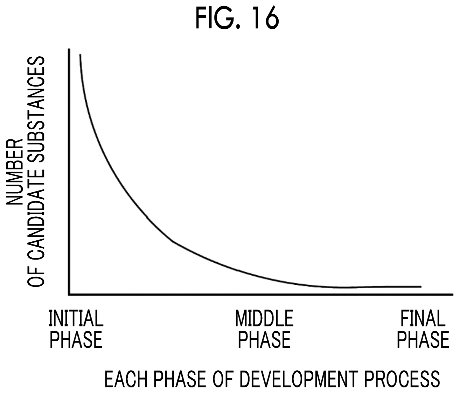

As shown in FIG. 16, in the development process of drug discovery, the number of candidate substances which are compounds to be predicted for physical properties is the largest in the initial phase of the development process, and decreases as the phase progresses to the middle or end phase. In a cloud system shared by a plurality of users 13, such as the physical property prediction system 11, a user 13 in charge of an initial phase and a user 13 in charge of a middle or final phase are mixed. The user 13 in charge of the initial phase inputs a large-scale prediction processing request as in the user 13A in many cases, and the user 13 in charge of the middle or final phase inputs a small-scale prediction processing request in many cases. The number of compounds as candidate substances for pharmaceuticals reaches, in the initial phase, several hundred thousand as an example. The candidate substances are narrowed down in the middle and final phases while the physical properties of the compounds of several hundred thousand are evaluated. Therefore, there is likely to be a large difference between the scale of the prediction processing request of the user 13 in charge of the initial phase and the scale of the prediction processing request of the user 13 in charge of the middle and final phases.

As in the control device 21 according to the second embodiment of the present disclosure, a technology having an effect of suppressing the waiting time of the user 13 who inputs a small-scale prediction processing request is particularly effective in a case where the number of the compounds to be predicted significantly fluctuates by the user 13, as shown in the field of drug discovery as an example.

In addition, in the second embodiment, the processor 41 sets the small-capacity queue 66 and the large-capacity queue 67 allowing parallel processing, and in the order control, the processor registers, among the large-scale job set of the user 13A, a part of the job set up to the threshold value in the small-capacity queue 66, registers the remaining part of the job set exceeding the threshold value in the large-capacity queue 67, and registers the small-scale job set of the user 13B in the small-capacity queue 66. As described above, since the order control is performed by setting two queues allowing parallel processing, it is possible to perform simple processing as compared with a case where the order control is performed by one queue.

In addition, in the second embodiment, as shown in FIG. 15, separate hardware resources 23A and 23B are assigned to the small-capacity queue 66 and the large-capacity queue 67. Therefore, it is easy to reduce the processing time as compared with a case where the job 61 registered in two queues of the small-capacity queue 66 and the large-capacity queue 67 is processed by one hardware resource 23.

### (Modification Example 1 of second embodiment)

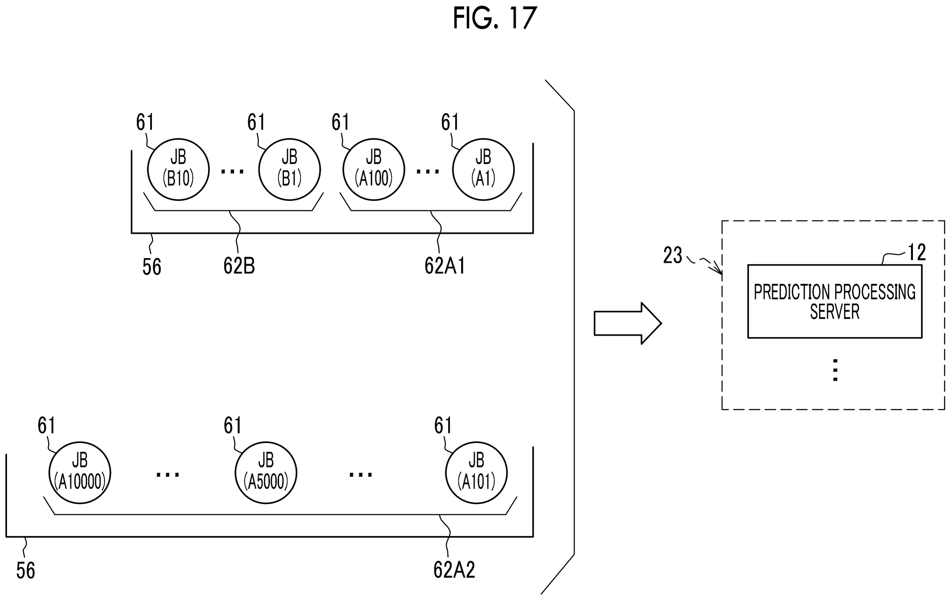

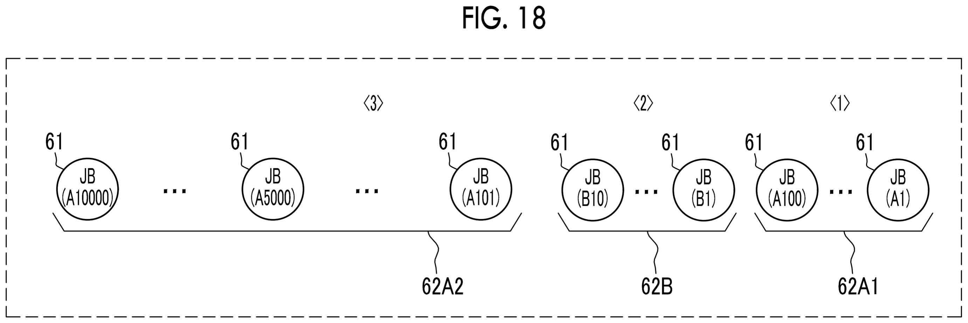

As shown in FIG. 17, separate hardware resources 23 may not be assigned to the small-capacity queue 66 and the large-capacity queue 67, or one hardware resource 23 may be used. In this case, as shown in FIG. 18 as an example, the hardware resource 23 executes the prediction processing in the order of the job set 62A1 of the user 13A registered in the small-capacity queue 66, the job set 62B of the user 13B registered in the small-capacity queue 66, and the job set 62A2 of the user 13A registered in the large-capacity queue 67. Even in this way, it is possible to perform the order control to give priority to the processing order of the small-scale job set of the user 13B.

### (Modification Example 2 of second embodiment)

In addition, in the above example, the example has been described in which the threshold value for determining the small-scale job set and the large-scale job set is the number of jobs, but the job score described in the first embodiment may be used as the threshold value instead of the number of jobs.

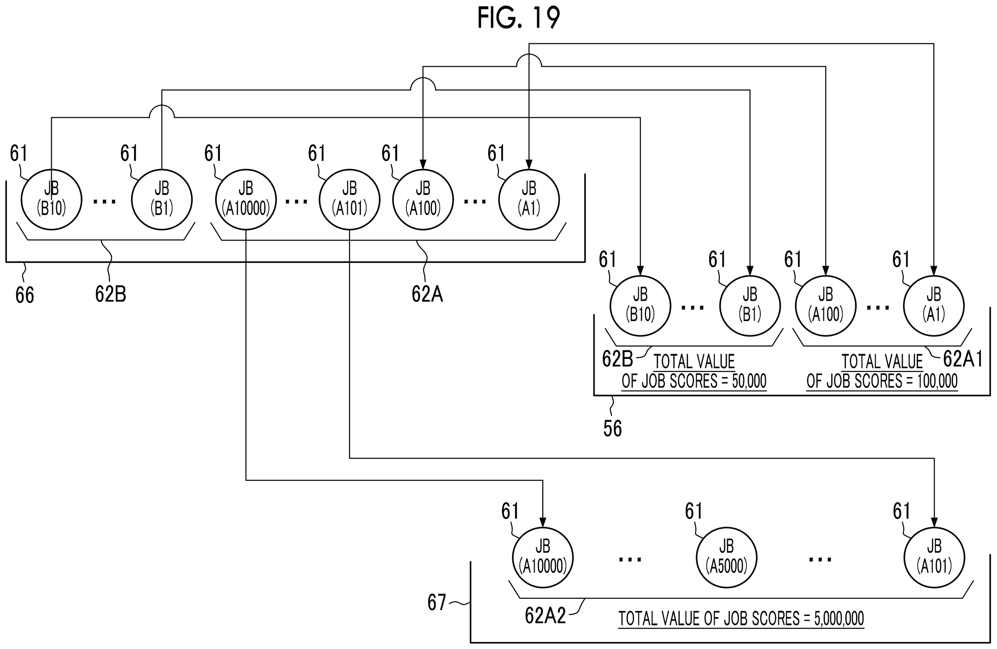

As shown in FIG. 19, the processor 41 derives the job score for the job set 62A of the user 13A and the job set 62B of the user 13B, compares the derived job score with the threshold value, and executes the job distribution processing of the small-scale job set and the large-scale job set.

The job score derivation processing in the first embodiment is performed to control the scale of the hardware resource 23. On the other hand, the job score derivation processing in Modification Example 2 of the second embodiment is performed for the job distribution processing according to the load. As described above, the job score derivation processing in Modification Example 2 of the second embodiment has a different purpose from the job score derivation processing of the first embodiment. In the case of the present example, since the job score derivation processing is performed in the job distribution processing, the processor 41 refers to the load function 57 in the job distribution processing to derive the job score.

In the example of FIG. 19, for example, the threshold value is "100,000" in the job distribution processing. The job score of the job set 62A of the user 13A is, for example, "5,100,000", and is determined to be a large-scale job set exceeding the threshold value. The processor 41 registers, in the small-capacity queue 66, a job set 62A1 having a job score of "100,000" which is equal to or less than the threshold value and which is a part of the job set 62A. Then, the processor 41 registers the remaining job set 62A2 having a job score of "5,000,000" in the large-capacity queue 67. In addition, the job score of the job set 62B of the user 13B is "50,000", and the processor 41 determines the job set 62B as the small-scale job set and registers the job set 62B in the small-capacity queue 66.

As shown in FIG. 19, in the small-capacity queue 66, the number of jobs in the job set 62A1 of the user 13A is 100, which is 10 times the 10 jobs in the job set 62B of the user 13B. However, in a case of comparing the job scores, which are loads in consideration of the molecular weight of the compound, the job score of the job set 62A1 is "100,000", which is twice "50,000" of the job set 62B. As described above, by using the job score as the threshold value, it is possible to more accurately estimate the load on the hardware resource 23. Since the load on the hardware resource 23 is related to the waiting time, the waiting time presented to the user 13 can be made close to the waiting time corresponding to the actual load.

In addition, in the above-described embodiment, the example in which the control device 21 is configured as an independent device different from the prediction processing server 22 has been described, but a form may be adopted in which the function of the control device 21 is incorporated in one or more prediction processing servers 22.

In addition, in the second embodiment, the scaling processing and the job distribution processing of the first embodiment are combined, but the job distribution processing may be performed without performing the scaling processing.

### (Appendices)

The following appendices can also be understood by the above description of the present disclosure.

### [Appendix 1]

A control device comprising a processor,
wherein the processor is configured to:
accept a request for prediction processing of predicting physical properties of a compound, the prediction processing request including compound information for specifying the compound to be predicted;
derive a job score that is an evaluation value indicating a magnitude of a load of a job corresponding to the accepted request, based on the compound information; and
control a scale of a hardware resource for executing a plurality of unprocessed jobs, based on the job score.

### [Appendix 2]

The control device according to Appendix 1,
wherein the processor is configured to acquire a correlation between the compound information and the load from a storage in which the correlation has been previously stored, and derive the job score.

### [Appendix 3]

The control device according to Appendix 1 or 2,
wherein the hardware resource is a server that executes the prediction processing, and
the processor is configured to determine, as the scale, the number of the servers to be assigned to the prediction processing.

### [Appendix 4]

The control device according to any one of Appendices 1 to 3,
wherein the processor is configured to, when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

### [Appendix 5]

The control device according to Appendix 4,
wherein the load is the number of the jobs or the job score.

### [Appendix 6]

The control device according to Appendix 4 or 5,
wherein the processor is configured to set, as queues for registering unprocessed jobs, two queues allowing parallel processing, which are a small-capacity queue in which the small-scale job set is registered and a large-capacity queue in which the large-scale job set is registered, and
in the order control, the processor is configured to:
   register, among the large-scale job set of the first user, a part of the job set up to the threshold value in the small-capacity queue;
   register the remaining part of the job set exceeding the threshold value in the large-capacity queue; and
   register the small-scale job set of the second user in the small-capacity queue.

### [Appendix 7]

The control device according to Appendix 6,
wherein separate hardware resources are assigned to the small-capacity queue and the large-capacity queue.

### [Appendix 8]

The control device according to any one of Appendices 1 to 7,
wherein the processor is configured to present a waiting time until completion of the job.

### [Appendix 9]

A method for operating a control device including a processor, the method comprising:
accepting, by the processor, a request for prediction processing of predicting physical properties of a compound, the request including compound information for specifying the compound to be predicted;
deriving, by the processor, a job score that is an evaluation value indicating a magnitude of a load of a job corresponding to the accepted request, based on the compound information; and
controlling, by the processor, a scale of a hardware resource for executing a plurality of unprocessed jobs, based on the job score.

### [Appendix 10]

An operation program for causing a computer to function as a control device, the operation program causing the computer to execute:
a process of accepting a request for prediction processing of predicting physical properties of a compound, the request including compound information for specifying the compound to be predicted;
a process of deriving a job score that is an evaluation value indicating a magnitude of a load of a job corresponding to the accepted request, based on the compound information; and
a process of controlling a scale of a hardware resource for executing a plurality of unprocessed jobs, based on the job score.

### [Appendix 11]

A physical property prediction system comprising:
the control device according to any one of Appendices 1 to 10; and
a prediction processing device that executes the prediction processing as the hardware resource.

### [Appendix 12]

A control device comprising a processor,
wherein the processor is configured to:
accept a request for prediction processing of predicting physical properties of a compound; and
when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

### [Appendix 13]

The control device according to Appendix 12,
wherein the load is the number of jobs for each unit of the compound or a job score that is an evaluation value indicating a magnitude of a load determined by the compound.

### [Appendix 14]

The control device according to Appendix 12 or 13,
wherein the processor is configured to set, as queues for registering unprocessed jobs, two queues allowing parallel processing, which are a small-capacity queue in which the small-scale job set is registered and a large-capacity queue in which the large-scale job set is registered, and
in the order control, the processor is configured to:
   register, among the large-scale job set of the first user, a part of the job set up to the threshold value in the small-capacity queue;
   register the remaining part of the job set exceeding the threshold value in the large-capacity queue; and
   register the small-scale job set of the second user in the small-capacity queue.

### [Appendix 15]

The control device according to Appendix 14,
wherein separate hardware resources are assigned to the small-capacity queue and the large-capacity queue.

### [Appendix 16]

The control device according to any one of Appendix 12 to 14,
wherein the processor is configured to present a waiting time until completion of the job.

### [Appendix 17]

A method for operating a control device including a processor, the method comprising:
accepting, by the processor, a request for prediction processing of predicting physical properties of a compound; and
when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, executing, by the processor, order control to give priority to a processing order of the small-scale job set of the second user over a processing order of a part of the large-scale job set of the first user.

### [Appendix 18]

An operation program for causing a computer to function as a control device, the operation program causing the computer to execute:
a process of accepting a request for prediction processing of predicting physical properties of a compound; and
when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, a process of executing order control to give priority to a processing order of the small-scale job set of the second user over a processing order of a part of the large-scale job set of the first user.

### [Appendix 19]

A physical property prediction system comprising:
the control device according to any one of Appendices 12 to 16; and
a prediction processing device that executes the prediction processing.

In addition, in the above-described embodiment, for example, as a hardware structure of the processor 41 that executes various types of processing such as the request acceptance processing, the job score derivation processing, the scaling processing, the waiting time presentation processing, and the job distribution processing, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific processing, such as an application specific integrated circuit (ASIC), in addition to a CPU that is a general-purpose processor configured to execute software (program) to function as various processing units.

Various types of processing described above may be executed by one of the various processors or may be executed by a combination of two or more processors (for example, a combination of a plurality of FPGAs or a CPU and an FPGA) of the same type or different types. A plurality of processing units may be configured by one processor. As an example in which the plurality of processing units are configured of one processor, there is a form in which a processor that realizes all functions of a system including the plurality of processing units by using one integrated circuit (IC) chip is used, such as a system on chip (SOC).

In this way, various processing units are configured by one or more of the above-described various processors as hardware structures.

Furthermore, the hardware structure of these various processors is, more specifically, an electric circuit (circuitry) in which circuit elements such as semiconductor elements are combined.

In addition, in addition to the operation program, the present disclosed technology also includes a non-transitory computer readable storage medium (a USB memory, a digital versatile disc (DVD)-read only memory (ROM), or the like) storing the operation program.

Contents described and illustrated above are for detailed description of a portion according to the technology of the present disclosure and are only an example of the technology of the present disclosure. For example, description related to the above configurations, functions, actions, and effects is description related to examples of configurations, functions, actions, and effects of the parts according to the disclosed technology. Accordingly, it is needless to say that unnecessary portions may be deleted, new elements may be added, or replacements may be made with respect to the content of the description made hereinbefore and the content of the drawings within a range that does not deviate from the gist of the present disclosed technology. In addition, in order to avoid complication and facilitate understanding of the parts according to the technology of the present disclosure, the description related to common technical knowledge or the like that does not need to be particularly described for enabling implementation of the technology of the present disclosure is omitted in the above-described contents and the above-shown contents.

In the present specification, "A and/or B" has the same meaning as "at least one of A or B". That is, "A and/or B" may be only A, only B, or a combination of A and B. In addition, in the present specification, a similar concept to "A and/or B" applies to a case where three or more matters are expressed by linking the matters with "and/or".

The disclosure of Japanese Patent Application No. 2023-099652 filed on June 16, 2023 is incorporated herein by reference in its entirety. In addition, all documents, patent applications, and technical standards described in the present specification are incorporated in the present specification by reference to the same extent as in a case where each document, patent application, and technical standard are specifically and individually noted to be incorporated by reference.

## Claims

1. A control device comprising a processor,
wherein the processor is configured to:
accept a request for prediction processing of predicting physical properties of a compound, the request including compound information for specifying the compound to be predicted;
derive a job score that is an evaluation value indicating a magnitude of a load of a job corresponding to the accepted request, based on the compound information; and
control a scale of a hardware resource for executing a plurality of unprocessed jobs, based on the job score.

2. The control device according to claim 1,
wherein the processor is configured to acquire a correlation between the compound information and the load from a storage in which the correlation has been previously stored, and derive the job score.

3. The control device according to claim 1,
wherein the hardware resource is a server that executes the prediction processing, and
the processor is configured to determine, as the scale, the number of the servers to be assigned to the prediction processing.

4. The control device according to claim 1,
wherein the processor is configured to, when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

5. The control device according to claim 4,
wherein the load is the number of the jobs or the job score.

6. The control device according to claim 4,
wherein the processor is configured to set, as queues for registering unprocessed jobs, two queues allowing parallel processing, which are a small-capacity queue in which the small-scale job set is registered and a large-capacity queue in which the large-scale job set is registered, and
in the order control, the processor is configured to:
register, among the large-scale job set of the first user, a part of the job set up to the threshold value in the small-capacity queue;
register the remaining part of the job set exceeding the threshold value in the large-capacity queue; and
register the small-scale job set of the second user in the small-capacity queue.

7. The control device according to claim 6,
wherein separate hardware resources are assigned to the small-capacity queue and the large-capacity queue.

8. The control device according to claim 1,
wherein the processor is configured to present a waiting time until completion of the job.

9. A method for operating a control device including a processor, the method comprising:
accepting, by the processor, a request for prediction processing of predicting physical properties of a compound, the request including compound information for specifying the compound to be predicted;
deriving, by the processor, a job score that is an evaluation value indicating a magnitude of a load of a job corresponding to the accepted request, based on the compound information; and
controlling, by the processor, a scale of a hardware resource for executing a plurality of unprocessed jobs, based on the job score.

10. An operation program for causing a computer to function as a control device, the operation program causing the computer to execute:
a process of accepting a request for prediction processing of predicting physical properties of a compound, the request including compound information for specifying the compound to be predicted;
a process of deriving a job score that is an evaluation value indicating a magnitude of a load of a job corresponding to the accepted request, based on the compound information; and
a process of controlling a scale of a hardware resource for executing a plurality of unprocessed jobs, based on the job score.

11. A physical property prediction system comprising:
the control device according to claim 1; and
a prediction processing device that executes the prediction processing as the hardware resource.

12. A control device comprising a processor,
wherein the processor is configured to:
accept a request for prediction processing of predicting physical properties of a compound; and
when, after accepting a request from a first user, accepting a request from a second user different from the first user, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, execute order control to give priority to a processing order of the small-scale job set of the second user over a processing order of a part of the large-scale job set of the first user.

13. The control device according to claim 12,
wherein the load is the number of jobs for each unit of the compound or a job score that is an evaluation value indicating a magnitude of a load determined by the compound.

14. The control device according to claim 12,
wherein the processor is configured to set, as queues for registering unprocessed jobs, two queues allowing parallel processing, which are a small-capacity queue in which the small-scale job set is registered and a large-capacity queue in which the large-scale job set is registered, and
in the order control, the processor is configured to:
register, among the large-scale job set of the first user, a part of the job set up to the threshold value in the small-capacity queue;
register the remaining part of the job set exceeding the threshold value in the large-capacity queue; and
register the small-scale job set of the second user in the small-capacity queue.

15. The control device according to claim 14,
wherein separate hardware resources are assigned to the small-capacity queue and the large-capacity queue.

16. The control device according to claim 12,
wherein the processor is configured to present a waiting time until completion of the job.

17. A method for operating a control device including a processor, the method comprising:
accepting, by the processor, a request for prediction processing of predicting physical properties of a compound; and
when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, executing, by the processor, order control to give priority to a processing order of the small-scale job set of the second user over a processing order of a part of the large-scale job set of the first user.

18. An operation program for causing a computer to function as a control device, the operation program causing the computer to execute:
a process of accepting a request for prediction processing of predicting physical properties of a compound; and
when, after accepting a request from a first user, a request from a second user different from the first user is accepted, in a case where a series of jobs corresponding to continuous requests from the first user is a large-scale job set having a load exceeding a preset threshold value and a series of jobs corresponding to continuous requests from the second user is a small-scale job set having a load equal to or less than the threshold value, a process of executing order control to give priority to a processing order of the small-scale job set of the second user over a processing order of a part of the large-scale job set of the first user.

19. A physical property prediction system comprising:
the control device according to claim 12; and
a prediction processing device that executes the prediction processing.
